# EUROPEAN PATENT APPLICATION

(11) **EP 4 614 144 A1**
(43) Date of publication of application: **10.09.2025**
(21) Application number: 24315080.2
(22) Date of filing: 05.03.2024
(51) Int. Cl.: G01N 33/50, G01N 33/543

(54) **METHOD FOR IMMUNODETECTION OF MEMBRANE AND CARGO PROTEIN MARKERS ON EXTRACELLULAR VESICLES**

(71) Applicant: Exosome Analytics, 91000 Evry-Courcouronnes (FR)
(72) Inventor: Khomyakova, Elena, 75015 Paris (FR)
(74) Representative: Yes My Patent

(57) **Abstract**

The invention relates to a method for the profiling of membrane and cargo proteins from extracellular vesicles (in combination or without simultaneous isolation of extracellular vesicles) consisting of a 3D ELISA technique, where the 3D support is beads placed in the wells of a microplate or in microtubes ("in the volume" reaction model) or on the surface of a filter membrane ("filtration" reaction model) and putted in contact with the extracellular vesicles.

## Description

[The invention relates to a method for the profiling of membrane and cargo proteins from extracellular vesicles (in combination or without simultaneous isolation of extracellular vesicles) consisting of a 3D ELISA technique, where the 3D support is beads placed in the wells of a microplate or in microtubes ("in the volume" reaction model) or on the surface of a filter membrane ("filtration" reaction model) and putted in contact with the extracellular vesicles.

### Technical Field

The invention relates to the field of immunodetection of membrane and cargo protein markers on extracellular vesicles.

### Background Art

A new approach in modern medicine is the concept of "liquid biopsy", where the molecular markers are analyzed in bio-fluid samples. Besides to circulated tumor cells and cell-free nucleic acids, extracellular vesicles are very promising in terms of liquid biopsy tests.

It is established that mammalian cells in their normal life and at various pathologies encapsulate specific components of their content into diverse populations of membrane vesicles generally named extracellular vesicles, or EVs. EVs are found in natural body fluids (blood, saliva, urine and breast milk) and in cell cultures. Extracellular vesicles are enriched in molecular markers (proteins, lipids, RNAs and dsDNA) specific for the cell of origin and involved in cellular processes including intercellular communications, immune-regulation and some others.

Extracellular vesicles attract enormous research interest because they are promising in important medical applications. The concentration of EVs in body fluids increases under different pathological conditions, including cancer. It was demonstrated that EVs participate in cancer progression and metastasis that makes them to be potentially perspective candidates for cancer liquid biopsy assays.

Much work has been focused on isolating the full spectrum of EVs (often small and medium EVs) from biological fluids and then analyzing their nucleic acids (namely miRNA and DNA) for biomarkers of different pathologies, particularly cancer. Despite the advantages of such approach, mainly due to high sensitivity of nucleic acids detection, the drawback of miRNA analysis is the absence of house-keeping exosomal miRNA identified at the moment. The main challenge while analyzing DNA mutational status is that usually wide spectrum of mutations is associated with similar tumor phenotypes that impede wide application of this method in liquid biopsy. However, recently it was demonstrated that isolation and quantification of the population of exosomes specific to a particular type of pathology by protein membrane markers is a simpler strategy that could see a quicker route to the clinic.

It is established that in addition to general membrane proteins markers such as CD81, CD9, CD63, exosomes carry the surface proteins specific to the cell emitter and thus can be considered as pathological markers. The main issue in isolation/characterization of pathology- related EVs by special markers is the fact that disease-related exosomes are just a small portion of general population of exosomes and they are shielded by serum/ascites/urine proteins. Moreover, the general markers are overrepresented at the surface of exosomes as compared to specific markers. Thus, the signals obtained from pathological EVs are usually orders of magnitude lower as compared to the signals that correspond to general exosomes markers (CD9, CD81, CD63), and for reliable quantification of pathological markers, sensitivity of detection method is crucial.

Three basic methods continue to be widely used for analysis of EVs membrane protein markers: Western Blotting, ELISA and Flow analysis of either immune complexes between EVs and antibody-coated beads or single vesicle profiling using high resolution flow cytometers.

Western blotting is semi-quantitative method dedicated for proteins detection and not suitable for their accurate quantification.

Flow analysis of EVs with application of magnetic or latex beads is widely used for quantification of specific EVs population. The approach is based on positive selection of target EVs population with antibody-coated beads followed by immunostaining of bead-bond vesicles using fluorescent labeled antibodies and flow analysis. Application of the beads allows obtaining highly accurate EVs transmembrane proteins quantification with good sensitivity. However, the sensitivity of the method is limited by the fact that EVs flow cytometry is based on analysis of single bead fluorescence and limit of detection (LOD) is crucially dependent on the ratio exosomes/ beads. Increasing EVs/beads ratio essentially increases signal/noise ratio. However, decreasing of beads amount is strongly decreasing the exosomes-beads binding kinetics similar to beads-cells reaction kinetics described in Shahid Waseem et al.Int. J. Mol. Sci. 2014, 15, 8821-8834; doi:10.3390/ijms15058821]. Both factors make flow analysis to be time consuming (at least overnight incubation of beads with EVs is required). Moreover, this approach demands expensive equipment and highly qualified staff.

ELISA is known to be a powerful tool for proteins quantification. However, non-specific binding is a well-known problem often observed in ELISA tests. Such non-specific binding can essentially increase the deviation of background signal that leads to decreasing of the method sensitivity. Moreover, since classical ELISA is 2-D assay and diffusion of exosomes, which are comparatively large, is much slower as compared to low molecular weight proteins, the sensitivity could be limited also by slow binding kinetics of low-represented population of vesicles.

Probably, insufficient sensitivity of widely used experimental techniques and insufficient purity of EVs samples are the main reasons why only a few works aimed at quantification of low-represented populations of EVs are published till present.

The present invention has been developed to overcome the whole drawbacks discussed above, and is presenting an innovative method for extracellular vesicles quantification and analysis thanks to a 3D ELISA technique.

### Summary of Invention

According to a first aspect, the present invention provides a method for the profiling of proteins from extracellular vesicles (EV) consisting of a 3D ELISA technique, characterized in that the 3D support are beads placed in the microplate wells or microtubes or on the surface of the filter membrane, putted in contact with the extracellular vesicles.

In some embodiments, said beads are magnetic or latex beads or polystyrene.

These beads have different surface functionalities.

In some embodiments, the beads have a size between 50 nm and 5 µm.

In some embodiments, the quantity of beads is between 10⁵ and 10⁸ per reaction.

In some embodiments, said beads are coated with crosslinking reagents that allows to immobilize the total extracellular vesicles population or to immobilize the entire set of proteins from lysed EVs.

In some embodiments, said crosslinking reagents are chosen among: Carbodiimide (e.g., EDC), NHS ester, Imidoester, Pentafluorophenyl ester, Hydroxymethyl phosphine, Maleimide, Haloacetyl (Bromo- or lodo-), Pyridyldisulfide, Thiosulfonate, Vinylsulfone, Hydrazide, Alkoxyamine, Diazirine, Aryl Azide, Isocyanate) capable of chemically attaching to specific protein reactive groups (Primary amines (-NH2); Carboxyls (-COOH); Sulfhydryls (-SH); Carbonyls (-CHO); epoxy groups.

In some embodiments, the pores of the filter membrane have a size between 5 kDa and 0.2 µm.

In some embodiments, the beads are coated with antibodies, that allows selection of total or specific populations of extracellular vesicles.

In some embodiments, the at least one antibody to general or specific protein markers is chosen among: CD9 or CD81 or CD63 or CD44 or CD10 or CD166 or CD117 or CD66 or CD147 or CD24 or EGFR or EpCam or CD105 or CA125 or NOTCH1 or NOTCH2 or NOTCH3 or CA15-5 or CA19-9 or PSA or PDL1 or PDL2.

In some embodiments, the method according to the invention comprises a step of removing the components which are unbound to the beads.

Method for the profiling of proteins from extracellular vesicles according to any one of the preceding claims, comprising a step of adding a labeling agent to visualize the beads-extracellular vesicles complexes.

In some embodiment, the said labeling agent is chosen among:
a. Fluorescent labeled antibodies to general or specific extracellular vesicles, preferably CD9 or CD81 or CD63 or CD44 or CD10 or CD166 or CD117 or CD66 or CD147 or CD24 or EGFR or EpCam or CD105 or NOTCH1 or NOTCH2 or NOTCH3 or NOTCH4 or CA125 or CA15-5 or CA19-9 or PSA or PDL1 or PDL2.
b. Lipid dyes, preferably DIO or DIL or DID; or
c. Nucleic acids staining dyes.

In some embodiments, the said step of removing the unbound labeling agent is made by sedimentation or by ultrafiltration or by centrifugation or using a magnet.

In some embodiments, the method comprises a step of registration for each sample in study of the total signal from the labeled EVs or cargo proteins attached to the beads.

In some embodiments, the said labelling agent could be at least one label-conjugated antibody to at least one specific membrane protein marker including: CD9 or CD81 or CD63 or CD44 or CD10 or CD166 or CD117 or CD66 or CD147 or CD24 or EGFR or EpCam or CD105 or NOTCH1 or NOTCH2 or NOTCH3 or NOTCH4 or CA125 or CA15-5 or CA19-9 or PSA or PDL1 or PDL2.

In some embodiments, the said antibody-conjugated labels could be Horseradish peroxidase (HRP) or alkaline phosphatase (AP) or beta-galactosidase (beta-gal).

In some embodiments, the said antibody-conjugated labels could be fluorescent dyes.

In some embodiments, the said labelling agent could be lipid dye including: DIO or DIL or DID.

In some embodiments, the said labelling agent could nucleic acids staining dye.

In some embodiments, at least one antibody specific to cytoplasmatic protein markers is chosen including: HSP70 or TSG101 or Alix or p53 or CD117 or CD166. In such case, extracellular vesicles should be lysed using one of common techniques (for example Kowel EJK et al.(2017) Extracellular vesicle isolation and analysis by western blotting. Methods Mol Biol. 1660:143-52).

In some embodiments, the method according to the invention comprises a step of removing the unbound labelling agent.

In some embodiments, the method according to the invention comprises a step of registration of the signal from the said labeling agent on the totality of the beads which follows labelling reaction.

In some embodiments, the extracellular vesicles are not pre-purified before being putted in contact with the beads using "in the volume" reaction model.

In such case the reaction of EVs-bead bonding could be carried our directly in cell culture supernatant or in body fluid after depletion of cells and cell debris.

Without disruption of the membrane of the pre-purified extracellular vesicles, the method according to the invention allows only to profile membrane proteins from the extracellular vesicles.

. In some embodiments, the extracellular vesicles are pre-purified before being putted in contact with the beads using "in the volume" reaction model.

In some embodiments, extracellular vesicles (pre-purified or not) are into contact with the beads by passing through a membrane on which the beads are placed (a "filtration" reaction model). This approach combines the steps of EV purification and profiling of protein markers.

In some embodiments, the extracellular vesicles are pre-purified by:
- differential centrifugation; or
- density gradients centrifugation; or
- ultrafiltration or
- ion column exchange; or
- immunoprecipitation with antibody-coated magnetic or latex beads; or
- polyethylene glycol (PEG) 8000 m precipitation.

In some embodiments, the membrane of the pre-purified extracellular vesicles is disrupted, preferably using a lysis buffer or/and ultrasound treatment, in order to extract and profile the cargo proteins from the extracellular vesicles (Kowel EJK et al. (2017) Extracellular vesicle isolation and analysis by western blotting. Methods Mol Biol. 1660:143-52).

Said disruption allows profiling both membrane and cargo proteins from the extracellular vesicles.

In some embodiments, the cargo proteins from lysed EVs are put in contact with beads coated with crosslinking reagents including Carbodiimide (e.g., EDC) or NHS ester crosslinking reagents (Carbodiimide (e.g., EDC) or NHS ester or Imidoester or Pentafluorophenyl ester or Hydroxymethyl phosphine or Maleimide or Haloacetyl (Bromo- or Iodo-) or Pyridyldisulfide or Thiosulfonate or Vinylsulfone or Hydrazide or Alkoxyamine or Diazirine or Aryl Azide or Isocyanate or epoxy groups capable of chemically attaching to specific protein reactive groups ( Primary amines (-NH2); Carboxyls (-COOH); Sulfhydryls (-SH); Carbonyls (-CHO) followed by immunostaining with antibodies to the cargo protein of interest conjugated with labelling agent(s). That allows visualization and quantification of the bead-protein complexes.

In some embodiments, extracellular vesicles are putted in contact with the beads by mixing them in solution using "in the volume" reaction model.

In some embodiments, extracellular vesicles are putted in contact with the beads by passing through the filter where the beads are placed on ("filtration" reaction model).

In some embodiments, said filters have the pore size between 10 nm and 1000 nm.

According to another aspect, the present invention provides the use of beads as a 3D support for an ELISA technique for profiling of EV proteins.

According to another aspect, the present invention provides a microplate for extracellular vesicles quantification and analysis comprising at least one row of wells, wherein antibody-coated or crosslinker-coated beads are placed in the plate wells.

According to another aspect, the present invention provides a method of diagnostic according to the invention to detect pathological protein markers on extracellular vesicles.

### Brief Description of Drawings

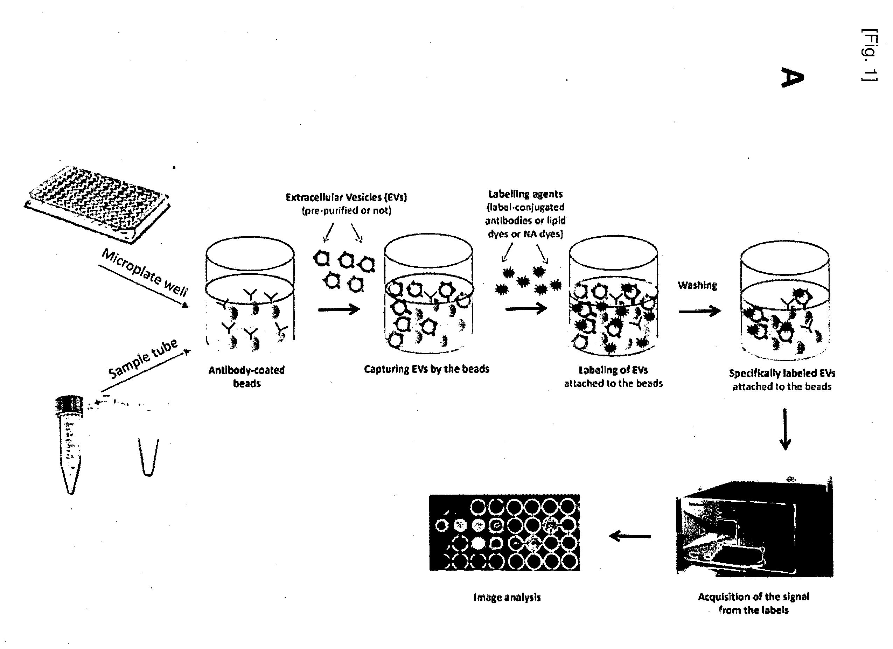

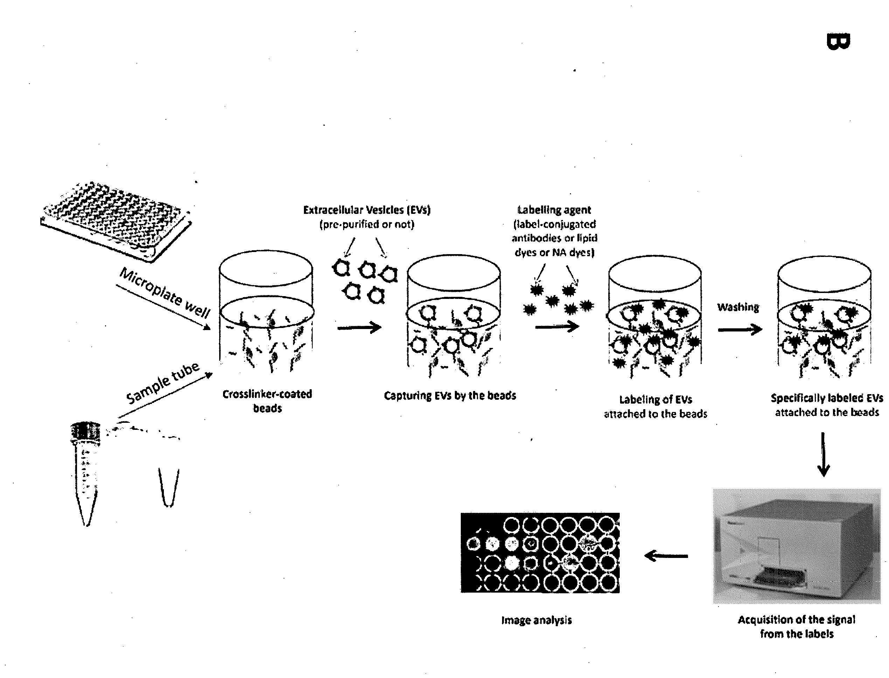

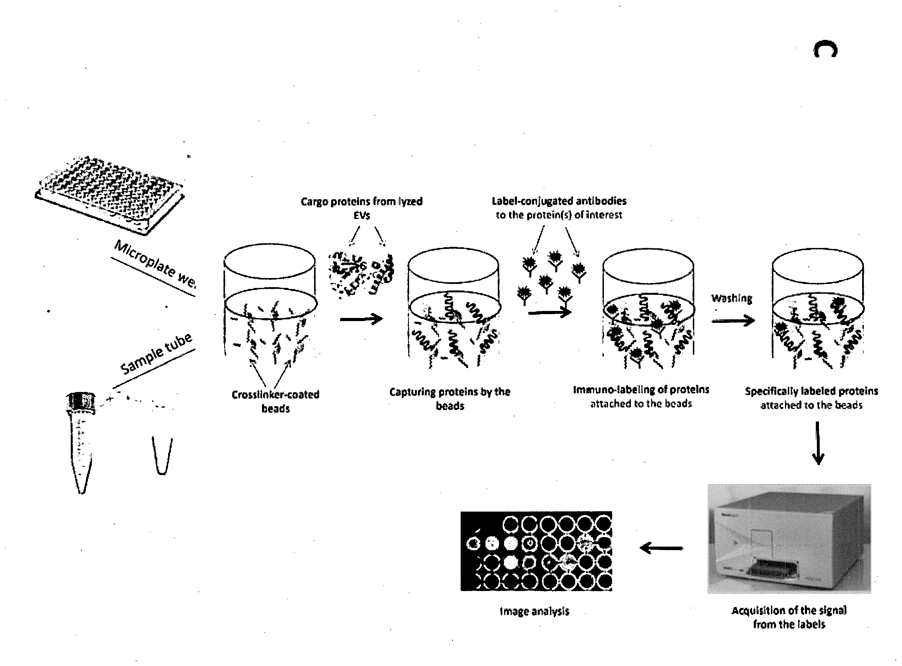

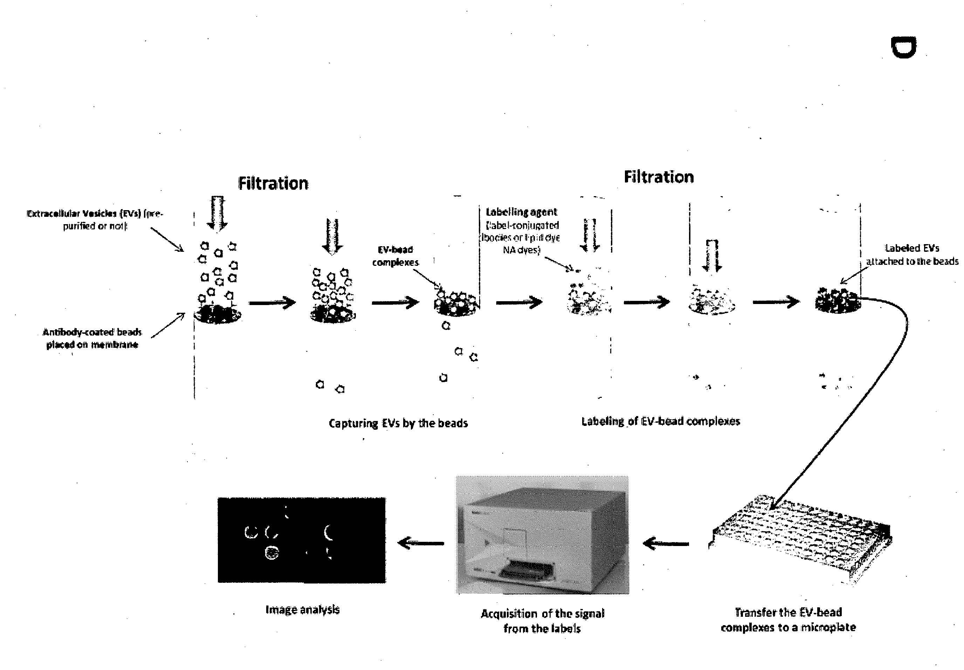

[Fig.1] shows the design of the 3D ELISA system according to the invention. In commonly used ELISA techniques, antibodies are attached to the surface of the wells. In our 3D ELISA system, antibodies are attached to beads which can move within the sample volume inside the microplate wells or microtubes, thereby accelerating the kinetics of the immune reaction ("in the volume" reaction model) or placed on the filter membrane ("filtration" reaction model). Another advantage of 3D ELISA technique is that beads coated with cross-linkers could be used. Such an approach allows capturing and analysis either total population of pre-purified EVs, or immobilization the totality of membrane proteins or cargo proteins (after lysis of extracellular vesicles). Fig. 1A: The scheme of 3D ELISA "in the volume" reaction model for profiling of membrane proteins on EVs while antibody(ies)-coated beads are used for the capture of extracellular vesicles. Fig. 1B: The scheme of 3D ELISA "in the volume" reaction model for profiling of membrane proteins on EVs while crosslinker-coated beads are used for the capture of extracellular vesicles. Fig. 1C: The scheme of 3D ELISA "in the volume" reaction model for profiling of cargo proteins on EVs while crosslinker-coated beads are used for the capture of cargo proteins from lyzed extracellular vesicles. Fig. 1 D: The scheme of 3D ELISA "filtration" reaction model for profiling of membrane proteins on EVs while antibody-coated beads are applied to the surface of a filter membrane through which extracellular vesicles pass.
[Fig.2] shows a Prototype of 3D ELISA system according to the invention "in the volume" reaction model.
[Fig.3] shows the quantification of 18 hours 3D ELISA fluorescent images (antiCD9-coated magnetic beads; anti-CD81-APC labelling of bead-attached EVs). Fig. 3A: 3D ELISA APC fluorescence image for different input of HT29 cell line-derived EVs . Fig. 3B: Quantification of fluorescence image. Fig. 3C. Dependence of Signal Noice Ratio on EVs input.
[Fig.4] shows the quantification 4 hours ELISA fluorescent images (antiCD9-coated magnetic beads; antiCD81-APC labelling of bead-attached EVs. Fig. 4A: 3D ELISA APC fluorescence image for different input of HT29 cell line-derived EVs input. Fig. 4B: Quantification of fluorescence image. Fig. 4C: Dependence of Signal Noice Ratio on EVs input.
[Fig.5] shows Quantification of 30 min 3D ELISA fluorescent images ("filtration" reaction model); antiCD9-coated magnetic beads; antiCD81-PE labelling of bead-attached EVs . Fig. 5A. 3D ELISA PE fluorescence image for different input of SKOV3 cell line-derived EVs . Fig. 5B. Quantification of fluorescence image. Fig. 5C. Dependence of Signal Noice Ratio on EV input.
[Fig.6] shows the detection of CD166 mesenchymal marker on EVs isolated from HEK293t cell culture ("in the volume" reaction model).
[Fig.7] shows the detection of CD44 mesenchymal marker on EVs isolated from HEK293t cell culture ("in the volume" reaction model).
[Fig.8] shows the detection of EpCam marker on EVs isolated from HEK293t cell culture ("in the volume" reaction model)..
[Fig.9] shows the detection of CA125 oncomarker on EVs isolated from HEK293t cell culture ("in the volume" reaction model).
[Fig.10] shows the detection of CA125 oncomarker on EVs isolated from SKOV3 cell culture («in the volume» reaction model).

### Description of Embodiments

The present invention enables the profiling of membrane proteins on EVs.

The present invention enables the profiling of cargo EV proteins.

Present invention enables simultaneous isolation of extracellular vesicles and profiling of membrane proteins on EVs ("filtration" reaction model).

The "enzyme-linked immunosorbent assay" or ELISA technique is a well-known technique for the person in the art, in which the recognition of an antigen under investigation by a specific antibody (or conversely of an antibody under investigation by an antigen) is followed by a reaction catalyzed by an enzyme which releases a colored component whose quantity is measured spectroscopically. For this purpose, the antigen is recognized by an antibody covalently coupled to an enzyme (or conversely the antibody is recognized by a labelled antigen).

Classical direct ELISA technique steps comprise several steps which could be resume as: the antigen is immobilized directly onto the surface of a multi-well microtiter plate such as a 96-well polystyrene plate, and then complexed with an enzyme-labeled primary antibody specific for the antigen. Once the enzyme-labeled primary antibody binds to the antigen, the conjugated primary antibody catalyzes a reaction with its respective substrate resulting in a visible colorimetric output that is measured by a spectrophotometer or absorbance microplate reader. Plate coating comprise the coating of an antigen in a plate.

As mentioned above, this technique has several drawbacks that the method according to the invention is overcoming, to provide a method for the profiling of proteins from extracellular vesicles.

The method according to the invention allows a dramatic acceleration of the kinesics of the reactions between extracellular vesicles and test reactive (antibody-coated beads, crosslinker-coated beads, labeled antibodies, lipids affinity dyes, nucleic acids binding dyes) through the use of beads as 3D support.

The design of the 3D ELISA system according to the invention is shown in the Figure 1. Figure 1A shows the option where extracellular vesicles from the sample in study are put in contact with antibody-coated beads for immune-selection of total or specific population of EVs (" in the volume" reaction model). Reaction duration could vary between 20 min and several days depending on EV quantity and membrane density of the membrane protein(s) used for positive selection. After that, labelling agent(s), including label-conjugated antibodies to general EVs markers (CD9, CD81 and CD63) or to the protein(s) of interest, or lipid dye(s), of nucleic acid dye(s) should be added to bead-EV complexes. Duration of immunolabeling reaction could vary from 15 min to several hours. One possibility is to add the labeling agent(s) directly to the reaction of the extracellular vesicles with the beads, which will increase the efficiency of EV labeling and reduce the overall assay time. Then, non-bound labeled antibody(ies) or another labeling agent(s) should be removed from the reaction mixture by sedimentation of bead-EVs complexes or using the magnet or by ultrafiltration followed by washing of labeled bead-EV complexes with PBS buffer supplied or nor with the detergent(s) to remove the label agents non-specifically attached to beads or to EVs. After the washing step, the total signal from the labels on the extracellular vesicles attached to the beads should be registered.

Second option is attachment of total population of extracellular vesicles from the sample in study to the crosslinker-coated beads using "in the volume" reaction model (Figure 1B). After removing of non-bond EVs by beads sedimentation or using magnet or with ultrafiltration, the bead-attached EVs should be stained with labelling agent(s) including by label-conjugated antibodies or lipid dyes or nucleic acids dyes. Then, non-bound labeled antibody(ies) or another labeling agent(s) should be removed from the reaction mixture by sedimentation of bead-EVs complexes or using the magnet or by ultrafiltration followed by washing of labeled bead-EV complexes with PBS buffer supplied or nor with the detergent(s) to remove the label agents non-specifically attached to beads or to EVs. After the washing step, the total signal from the labels on the extracellular vesicles attached to the beads should be registered.

The third possibility (Figure 1C) is lysis of preferably pre-purified extracellular vesicles from the sample in study and putting the cargo and membrane proteins from lysed EVs in contact with crosslinker-coated beads using "in the volume" reaction model. After that, the non-bound proteins should be removed from bead-protein complexes by beads sedimentation or using magnet or by ultrafiltration and by label-conjugated antibody(ies) to the protein(s) of interest should be added to bead-protein complexes. Duration of labeling reaction depends on quantity of the protein(s) of interest and could vary from 15 min to several hours. Non-bound label-conjugated antibody(ies) should be removed from the reaction mixture by sedimentation of bead-protein complexes or using the magnet or by ultrafiltration followed by washing of labeled bead-protein complexes with PBS buffer supplied or nor with the detergent(s) to remove the label agents non-specifically attached to beads or to bead-attached proteins. After the washing step, the total signal from the labeling agents reacted with the proteins attached to the beads should be registered.

"in the volume"reactions of EVs/bead bonding carries either on the rotator or without rotation (in case of small nanosized beads).

Profiling of membrane protein markers on extracellular vesicles could be combined with EVs isolation (Figure 1,D). Non-purified or dissolved in large volume pre-isolated EVs should pass through the filter membrane on which the antibody-coated beads are places on. The pores of the filter membrane could be either larger than the size of the EVs (for example, 0.2 µm) allowing extracellular vesicles to pass through the membrane, or smaller than the size of the EVs (for example, 1000 kDa or 500 kDa or 300kDa or 100 kDa or 50 kDa or 10 kDa or 5 kDa) allowing to concentrate EVs close to the membrane surface. Under gravity or pressure or centrifugation force, the EVs-containing solution passes through the filter membranes allowing the EVs to be captured by the beads using antigen-antibody interaction following EVs labeling using similar procedure. After that the labeled bead-EV complexes should be collected from the membrane surface, washed from non-bound label and analysed as shown in Figure 1,D.

Profiling of membrane protein markers on extracellular vesicles can be combined with isolation of EVs (Figure 1D). Non-isolated or pre-isolated EVs dissolved in large volume of appropriate buffer should be passed through a filter membrane on which antibody-coated beads are placed by gravity, pressure, or centrifugal force,. The pores of the filter membrane can be either larger than the size of the EVs (for example, 0.2 µm) allowing extracellular vesicles to pass through the membrane, or smaller than the size of the EVs (for example, 1000 kDa, or 500 kDa, or 300 kDa, or 100 kDa or 50 kDa or 10 kDa or 5 kDa), that allows concentration of EVs in a small volume close to the membrane surface.

By passing through a filter membrane or being concentrated close to the membrane surface, EVs will be captured by the beads using antigen-antibody interaction following labeling the EVs using a similar procedure. Labeled bead-EV complexes should then be collected from the membrane surface, washed to remove unbound label, and analyzed as shown in Figure 1 D.

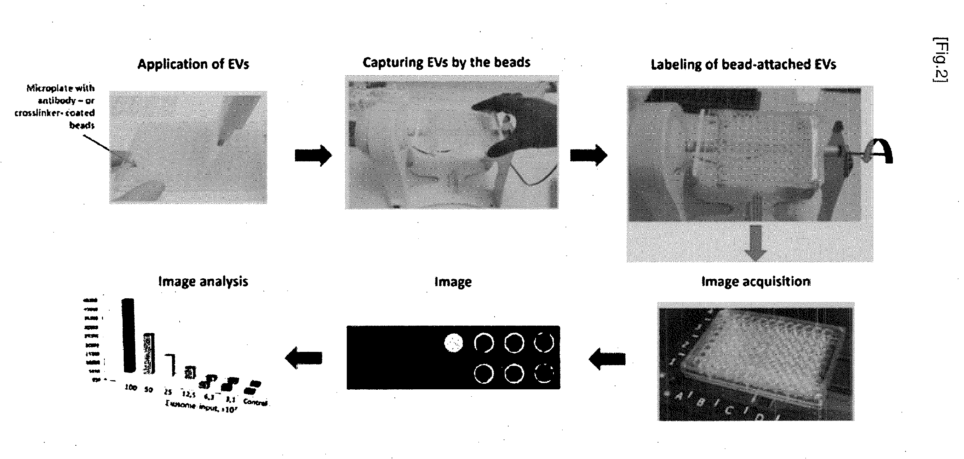

Figure 2 is a photo of the 3D ELISA prototype. Contrary to classic ELISA where test antibodies are attached to the microplate wells surface, in the present design antibody- or crosslinker coated beads are used as a 3D support for attaching of total (CD9/CD81/CD63 positive) or specific EV populations or EV cargo proteins (Figure 1). In present design the beads are placed in microplate wells, but the reactions could be carried out in microtubes as well. The reaction of EVs/bead bonding carries either on the rotator or directly on the bench (in case of small nanosized beads).

The 3D bead-based ELISA system according to the invention allows profiling of EV cargo proteins (which reflect the spectrum of cytoplasmic proteins of the cell of origin). In such case, the membrane of preferably pre-purified EVs (by differential centrifugation/ by density gradients centrifugation/ by ultrafiltration/by ion column exchange or by immunoprecipitation with antibody-coated magnetic or latex beads) must be disrupted using the lysis buffer and/or ultrasound treatment or beads homogenization to obtain the pull of INTRA-vesicle cargo proteins.

Analysis and quantification of the obtained images. The TOTAL signal from total beads population should be registered. The signal from labeled EVs attached to the beads is considered as "specific signal". Depending on the application, the background signal could be subtracted (for example, signal from EVs attached to the beads and immunolabeled with isotype control to the antibody of interest, or signal from beads without EVs stained with antibody of interest) .

The advantage of bead-based 3D ELISA over classic ELISA assays is in (1) very low non-specific binding of the labelling agents to the beads; (2) essentially faster kinetics of EVs - beads binding through beads mixing withing the reaction volume and (3) larger total surface of the beads participating in the reaction as compared to the surface of microplate well bottom what allows immobilizing larger quantity of antibodies or crosslinkers as well as (4) possibility the to profile protein markers in low-concentration solutions containing EVs or proteins using a filtration set-upThe advantage 3D bead-based ELISA over classical flow cytometry analysis of EV-beads complexes is that registration of the signal from the label used performs from the totality of the beads surface for each microplate well. Due to this, to increase the signal corresponding to single bead it is not necessary to decrease the number of beads, as for flow cytometry analysis. Opposite, the increasing of beads quantity leads to increasing the kinetics of the reactions between beads and extracellular vesicles or cargo proteins and between bead-EV or bead-protein complexes that allows decreasing the reactions duration and improving the sensitivity of the method.

The fluorescence was registered with Eppendorf Typhoon Biomolecular imager and analyzed either with Image-J software or home- made image analysis software.

### Definitions

Extracellular vesicles (EVs) are lipid bound vesicles secreted by cells into the extracellular space. They have been attributed to roles in cell-cell communication, cancer metastasis, and early disease diagnostics (Daaboul et al. 2016). EVs population (or Exosomes) is divided to large, middle and small (including exosomes) vesicles. Different sub-populations of EVs including large apoptotic bodies, shedding vesicles formed by cell membrane blebbing and exosomes are distinguished at present. Exosomes, the most studied vesicles population, are small (40-100 nm) vesicles of endocytic origin. Since in present no markers are defined to distinguish exosomes from small (fewer than 100 nm) membrane blebbing vesicles, new classification of EVs was proposed [Théry C, Witwer KW, Aikawa E, Alcaraz MJ, Anderson JD, Andriantsitohaina R. et al. Minimal information for studies of extracellular vesicles 2018 (MISEV2018): a position statement of the international society for extracellular vesicles and update of the MISEV2014 guidelines. J Extracell Vesicles. 2018;7:1535750). According to MISEV2018, EVs can be divided into medium/large EVs (>200 nm) and small EVs (<200 nm) based on their physical properties of EVs. EVs can also be classified into apoptotic bodies (50-1000 nm in diameter), microvesicles (MVs) (100-1000 nm), and exosomes (40-160 nm, average- 100 nm) based on their origin.

By "profiling of proteins" is intended according to the invention that the method allow to identify, quantify and analyze the proteins from the studied extracellular vesicles.

By 3D bead-based ELISA (or 3D ELISA), according to the invention, is intended that the totality of the beads used as a support for extracellular vesicles or EV cargo proteins attachment have an increased surface area as compared to plate well. For example, while 10⁶ of 4.5 µm beads used in the reaction, the total beads surface is 8.5 times larger than the surface of the bottom of 96-well plate; while 10⁷ 4.5 µm beads used in the reaction, the total beads surface is about 85 times larger than the surface of the bottom of 96-well

By "beads" is intended according to the invention these mostly spherical particles, preferably of a size between 50 nm to 5 µm.

Magnetic beads are made of nanometric-sized iron oxide particles encapsulated or glued together with polymers.

Latex beads are formed from amorphous polymer (usually polystyrene).

By "put in contact" is intended according to the invention that for example the EVs are added into the microplate after the beads have been, in order to provoke a liaison between the antibody attached to the beads (or the epoxy beads) and the EVs.

By "coating" or "coated" is intended according to the invention the attachment of antigens, antibodies or other compounds to the surface of the beads.

By "epoxy" is intended according to the invention is intended according to the invention that the beads have epoxy groups on the surface. This beads covalently bind to the primary amine and sulfhydryl groups of proteins and peptides. An example of such epoxy beads is Dynabeads^{™} M-270.

By "total population" of EVs, according to the invention is meant that the entire population of EVs of the sample studied is targeted by the method.

By "specific population" of EVs is intended according to the invention that only some of the EVs that carry specific protein markers are targeted by the method. Example of said specific markers are CD9 or CD81 or CD63 or CD44 or CD10 or CD166 or CD117 or CD66 or CD147 or CD24 or EGFR or EpCam or CD105 or NOTCH1 or NOTCH2 or NOTCH3 or NOTCH4 or CA125 or CA15-5 or CA19-9 or PSA or PDL1 or PDL2.

"Cargo proteins" define any protein that is carried within the vesicles of a cell's secretory system.

CD9 is a gene encoding a protein that is a member of the transmembrane 4 superfamily also known as the tetraspanin family. It is a cell surface glycoprotein that consists of four transmembrane regions and has two extracellular loops that contain disulfide bonds which are conserved throughout the tetraspanin family. CD9 is commonly used as a marker for exosomes as, it is contained on their surface. CD9 can also modulate cell adhesion (*Machado-Pineda Y, et al 2018*) and migration (Blake DJ, et al. 2018 & Miki Y., et al, 2018) This function makes CD9 of interest when studying cancer and cancer metastasis. However, it seems CD9 has a varying role in different types of cancers. Studies showed that CD9 expression levels have an inverse correlation to metastatic potential or patient survival. The over expression of CD9 was shown to decrease metastasis in certain types of melanoma, breast, lung, pancreas and colon carcinomas(Mimori K., et al. 1998 & Higashiyama M., et al. 1995).

CD81 is a gene encoding a protein which is a member of the transmembrane 4 superfamily, also known as the tetraspanin family. This cell surface glycoprotein mediates signal transduction events that play a role in the regulation of cell development, activation, growth and motility.

CD63 is a cell surface glycoprotein that is known to complex with integrins. CD63 is also a member of the tetraspanin family. It may function as a blood platelet activation marker. As CD81, CD63 mediates signal transduction events that play a role in the regulation of cell development,.activation, growth and motility.

CD44 is a cell-surface glycoprotein involved in cell proliferation, cell differentiation, cell migration, angiogenesis, presentation of cytokines, chemokines, and growth factors to the corresponding receptors, and docking of proteases at the cell membrane, as well as in signaling for cell survival. This protein has been showed to be implicated in various pathologie (leukemia, cancers (renal cancer and non-Hodgkin's lymphomas are exceptions)..).

EpCam, also known as CD326, is a transmembrane glycoprotein mediating Ca2+-independent homotypic cell-cell adhesion in epithelia. EpCAM is also involved in cell signaling, migration, proliferation, and differentiation. EpCAM is expressed exclusively in epithelia and epithelial-derived neoplasms, and can be used as diagnostic marker for various cancers.

PSA or Prostate-specific antigen, also known as gamma-seminoprotein or kallikrein-3 (KLK3), is a glycoprotein enzyme, member of the kallikrein-related peptidase family, which is secreted by the epithelial cells of the prostate gland. Evaluating the levels of PSA helps to diagnose prostate cancer.

CD105 or Endoglin is a type I membrane glycoprotein located on cell surfaces, part of the TGF beta receptor complex. It has a crucial role in angiogenesis, making it an important protein for tumor growth, survival and metastasis of cancer cells to other locations in the body.

CD10, also known as Common Acute Lymphocytic Leukemia Antigen (CALLA), is a cell surface enzyme that is encoded by the human gene MME (Membrane Metalloendopeptidase). CD10 is a metalloprotease that belongs to the neutral endopeptidase family. It is commonly found on the surface of certain cells, including lymphocytes and some types of tumor cells.

CD166, also known as activated leukocyte cell adhesion molecule (ALCAM), is a protein that in humans is encoded by the ALCAM gene. It is a cell adhesion molecule belonging to the immunoglobulin superfamily. CD166 is a transmembrane glycoprotein, meaning it spans the cell membrane and has portions both inside and outside the cell.

CD117, also known as c-kit or stem cell factor receptor, is a protein that is encoded by the KIT gene in humans. It belongs to the type III receptor tyrosine kinase family and plays a crucial role in the regulation of cellular processes, including cell survival, proliferation, and differentiation.

CD66 refers to a family of cell surface glycoproteins that are members of the carcinoembryonic antigen-related cell adhesion molecule (CEACAM) family. CEACAM proteins are involved in cell adhesion, immune response, and signal transduction. CD66 includes various isoforms, such as CD66a, CD66b, CD66c, CD66d, CD66e, and others. One well-known member of the CD66 family is CD66b, also called CEACAM8 or NCA-95. CD66b is primarily expressed on the surface of neutrophils, which are a type of white blood cell involved in the immune response. It plays a role in neutrophil adhesion and migration, contributing to the immune system's ability to respond to infections and inflammation.

CD147, also known as Basigin or EMMPRIN (extracellular matrix metalloproteinase inducer), is a transmembrane glycoprotein that belongs to the immunoglobulin superfamily. The CD147 protein is encoded by the BSG gene in humans.

CD24 is a cell surface protein that is encoded by the CD24 gene in humans. It is a small glycosylphosphatidylinositol (GPI)-linked protein, meaning it is anchored to the cell membrane by a GPI anchor. CD24 is expressed in various tissues and cell types, and its expression can be regulated during development, differentiation, and in response to different physiological conditions.

The EGFR protein is a transmembrane glycoprotein that is a member of the protein kinase superfamily. This protein is a receptor for members of the epidermal growth factor family. EGFR is a cell surface protein that binds to epidermal growth factor, thereby causing receptor dimerization and tyrosine autophosphorylation, leading to cell proliferation. Mutations in this gene are associated with lung cancer.

CA125 or cancer antigen 125, is a protein that is often used as a tumor marker to monitor certain types of cancers, particularly ovarian cancer. The CA125 protein is encoded by the MUC16 gene in humans.

CA15-5 is a high molecular weight glycoprotein (300-450 kDa). It can be increased in some cancerous pathologies: breast, ovarian, liver and sometimes lung cancers. Its level can also be modified in people who suffer from so-called autoimmune diseases or chronic hepatitis. CA15-5 is present in the blood in significant quantities in 30 to 50% of women with breast cancer and could be used for evaluation of therapeutic effectiveness, monitoring and detection of cancer recurrence.

CA19-9, or carbohydrate antigen 19-9, is a tumor marker that is often used in the context of gastrointestinal cancers, especially pancreatic cancer. It is a glycoprotein, and elevated levels of CA19-9 in the blood can be associated with certain malignancies.

Programmed Death-Ligand 1 (PD-L1) is a cell surface protein that plays a crucial role in the regulation of the immune system. It is commonly associated with cancer and immunotherapy.

Programmed Death-Ligand 2 (PDL2), and it is a cell surface protein that plays a role in regulating the immune system. PD-L2 is a member of the B7 family of proteins, and its primary function is to interact with its receptor, PD-1 (Programmed Cell Death Protein 1), on T cells.

The Notch signaling is a highly conserved cell signaling pathway which is responsible for regulation of self-renewal and differentiation in several tissues and cell types. Notch is a binary cell-fate determinant. Mammals possess four different notch receptors, referred to as NOTCH1, NOTCH2, NOTCH3, and NOTCH4. Notch signaling is dysregulated in many diseases including many cancers.

By "removing" or "washing" is intended according to the invention that any unbound component is removed from bead-EV or bead-protein complexes

By "unbound components" is intended according to the invention the extracellular vesicles or the proteins, or labeled agents that are not bound to the beads or to bead-EV complexes or to bead-protein complexes and could not be study in the technique according to the invention. Preferably, removing the unbound markers is made by sedimentation or by using a magnet in case of magnetic beads or by ultrafiltration in case of "in the volume" reaction model or by filtration in "filtration" reaction model.

By "components which are unbound to the beads" are intended the unbound EVs, the unbound cargo proteins, the unbound antibodies, and/or the unbound labeling agents.

By "a fluorescent marker" is intended according to the invention specific molecules, like protein, which are covalently bound fluorophores that selectively bind to a functional group of the target for detection. The most commonly used fluorescent molecules are antibodies. Here, these markers allow to visualize the extracellular vesicles attached to the beads

"Fluorescent labeled antibodies" are antibodies that are coupled with fluorescent moieties.

"Lipid dyes" are dyes that binds to lipids.

DiO also called DiOC₁₈(3), is a green fluorescent, lipophilic carbocyanine dye that is widely used as a lipophilic tracer.

DIL also known as DiIC18(3), is a fluorescent lipophilic cationic indocarbocyanine dye and indolium compound, which is usually made as a perchlorate salt.

DID or (DiIC18(5); 1,1'-dioctadecyl-3,3,3',3'- tetramethylindodicarbocyanine, 4-chlorobenzenesulfonate salt) is a lipophilic carbocyanine dye.

"Nucleic acids staining dye" is a sensitive fluorescent dye that binds to single-stranded DNA, double-stranded DNA and RNA or DNA/RNA intercalating dyes and can be used to stain and visualize nucleic acids.

By "pre-purified" is intended according to the invention that the EVs are purified before the first step of contact with the beads. This step of pre-purification is made by any method included, differential centrifugation; or density gradients centrifugation; or ultrafiltration; or ion column exchange; or immunoprecipitation with antibody-coated magnetic or latex beads.

Centrifugation allows to separate substances of different size and density by means of centrifugal force.

Density gradients centrifugation is a method where the components of a sample are separated on the basis of their density, in a dense medium or density gradient, in a centrifuge, according to the centrifugal force they experience.

Sedimentation refers to the process by which compounds settle or separate from a solution under the influence of gravity or centrifugal force. This is a known laboratory techniques, to isolate or analyze proteins based on their sedimentation rates.

Ultrafiltration is a membrane separation method, which the size of the retained particles is between 10³ and 10⁶ Dalton (Da)

Ion column exchange is a technique consisting of a tube packed with particles or beads of resin chosen for their ability to capture specific ions from an aqueous solution as it passes through the column.

A lysis buffer is buffer solution used for the purpose of breaking EVs to have an access to their cargo proteins.
The step of registration of the total signal from the label used is registration of total signal from each microplate well containing sample to be analyse. Mean or median signal form total surface of each well should be taken into account. To calculate the "specific signal", the subtraction of background signal should be realized. EV-free beads labeled with the same label as bead-EV complexes or bead-EV complexes labeled with isotype control to the antibody of interest could be used as a background signal.

In the description and in the following examples, unless otherwise indicated, percentages are percentages by weight and ranges of values denominated as "between ... and ..." include the lower and upper limits specified.

The following examples are illustrative and not limiting of the scope of the invention.

### Examples

### Example 1: Evaluation of the sensitivity of antibodies-based 3D ELISA assay and comparison its sensitivity to classic ELISA tests applied for EVs analysis. Experimental design

This study was run to evaluate the sensitivity of the 3D ELISA assays according to the invention, and to compare its sensitivity to classic ELISA tests applied for EVs analysis.

Extracellular vesicles (EVs) were isolated from HT29 human colorectal adenocarcinoma cell culture supernatant or HEK293t immortalized human embryonic kidney cell culture supernatant or from SKOV3 human ovarian cancer cell culture supernatant. The concentration of isolated EVs was measured by NTA as described in (M. Livshits et al. Sci Rep 2015 Nov 30:5:17319. doi: 10.1038/srep17319.). 2,7 µm antibody magnetic beads were used for immunoselection of pre-purified small EVs. Incubation time was either 2 hours or overnight. EVs-bead complexes were stained with different combination of antibodies as mentioned in the figures. The fluorescence was registered with Eppendorf Typhoon Biomolecular imager and analyzed either with Image-J software or home- made image analysis software.

To estimate the sensitivity of 3D ELISA assay, Signal/Noise Ratio (SNR) was calculated for each EV input. In present study the "Signal" was considered as difference between the signal form labeled EVs attached to the beads after subtraction of background (non-specific labeling of beads without EVs). Noise is Standard deviation of background value calculated for at least 3 independent background samples. The Limit of Detection (LOD) of the assay was determined as EV input value corresponding to SNR=2. The sensitivity of 3D ELISA assay as compared to the data of the competitors (System Biosciences) represented in the site of the Company.

### Results

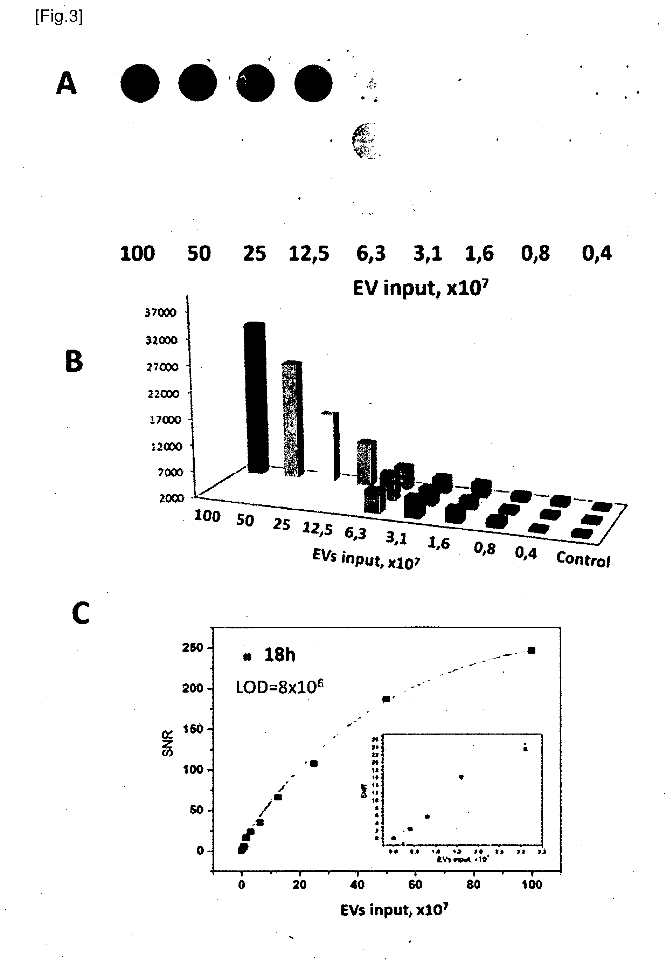

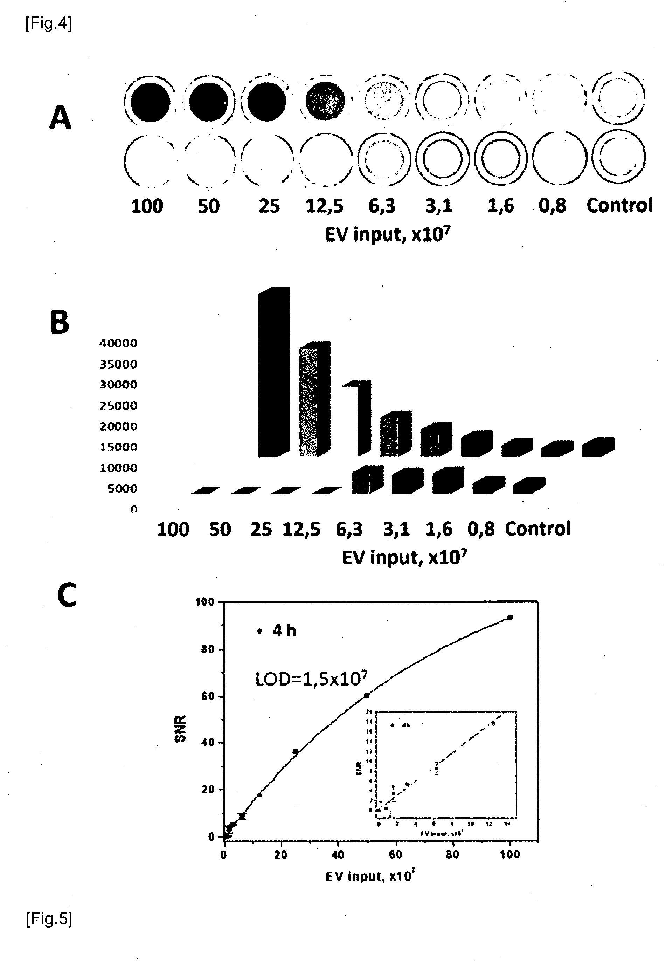

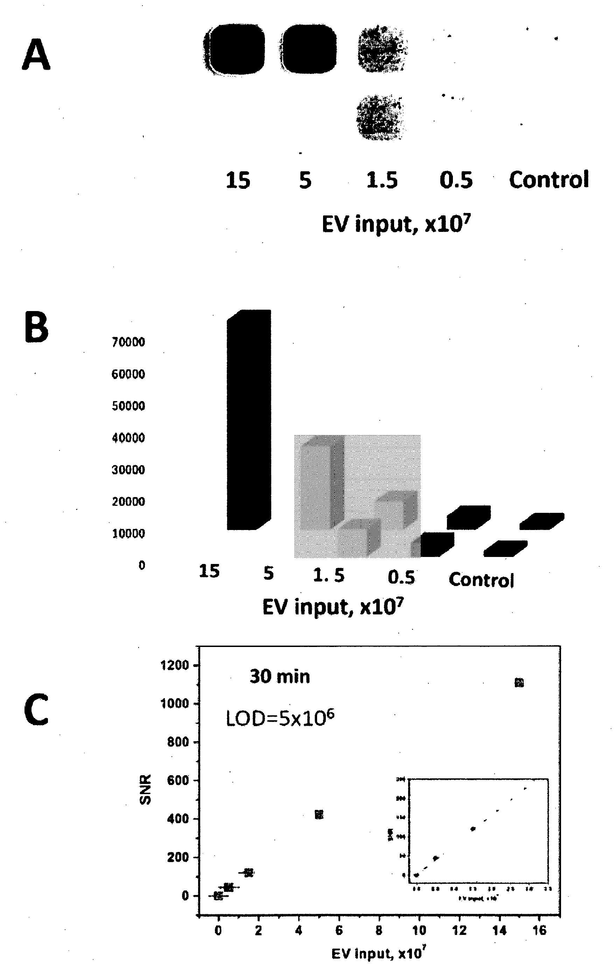

Cell culture. The fluorescent images of microplate wells for 18 h and 4 h experiment durations ("in the volume" reaction model) and for 30 min ("filtration" reaction model) are shown in the Figures 3A, 4A and 5A correspondently. Figures 3B, 4B and 5B represent the graphs of total median fluorescent signals values corresponding to each microplate well at different EVs input. The curves representing the dependence of Signal Noise Ratio (SNR) on EVs input for 18 hours, 4 hours and 30 min of test duration are shown in Figures 3C, 4C and 5C correspondently. As follows from the graphs, limit of detection (LOD) of bead-based 3D ELISA for "in the volume" reaction setup is 8×10⁶ for CD9⁺/CD81⁺ EVs for 18 hours of reaction duration and 3×10⁷ for CD9⁺/CD81⁺ EVs in case of 4 hours of reaction duration As follows from the data, "filtration" reaction setup provides an advantage in the sensitivity of detection of protein markers on EVs (LOD is 5×10⁶ for CD9⁺/CD81⁺ EVs ). Bead-based 3D ELISA applied for extracellular vesicles analysis is about 2 orders of magnitude more sensitive (3×10⁷ vs ~4×10⁹ ) than classical ELISA tests proposed by competitors (for example, ExoELISA-ULTRA Complete Kit (CD81 Detection) by System Biosciences; https://www.systembio.com/exo-elisa-ultra-complete-kit-cd81-detection). Such gain in sensitivity of bead-based 3D ELISA over conventional ELISA assays applied for analysis of EVs is due to very low non-specific binding of labeled antibodies to the beads, essentially quicker kinetics of EV capturing by antibody-coated beads equally distributed withing the reaction volume and by larger total surface of the beads than the surface of 96 plate bottom.

The high sensitivity of the 3D ELISA technique (even the "in the volume" reaction model) allows its application for highly sensitive profiling of low-represented protein markers on extracellular vesicles. While using antibody-coated beads, two approaches could be used: (1) capturing of EVs using the beads coated with antibodies to the marker(s) of interest followed by labelling of beads-attached EVs using labeled antibodies to general EV markers (CD9 or CD81 or CD63) or with labeled antibodie(s) to the protein(s) of interest or with lipid dyes or with nucleic acids staining dyes as described in the invention , or (2) capturing of EVs with the beads coated with antibodies to general EV markers 'CD9 or CD81 or CD63) followed by labelling of bead-attached EVs with labeled antibodie(s) to the protein(s) of interest or with lipid dyes or with nucleic acids staining dyes as described in the invention

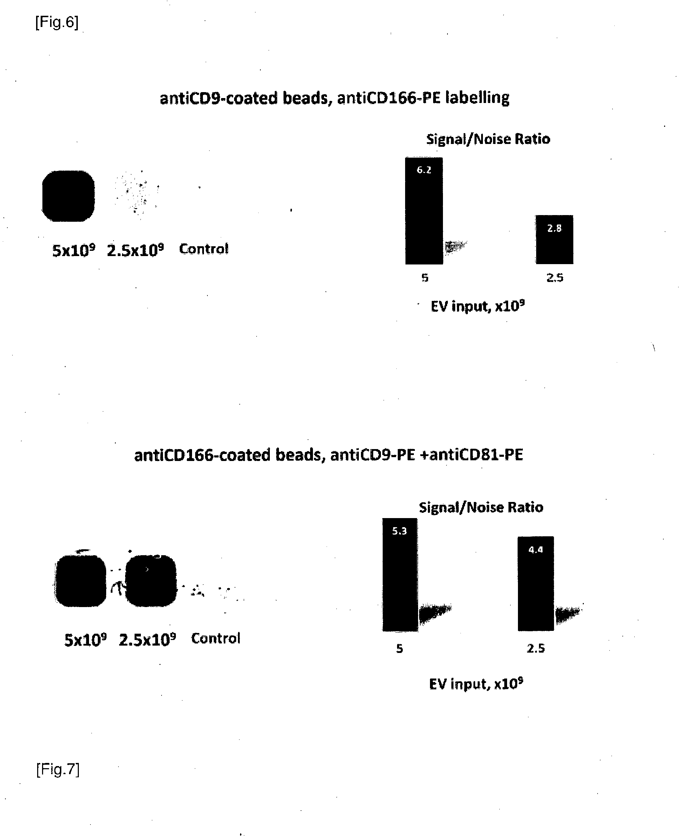

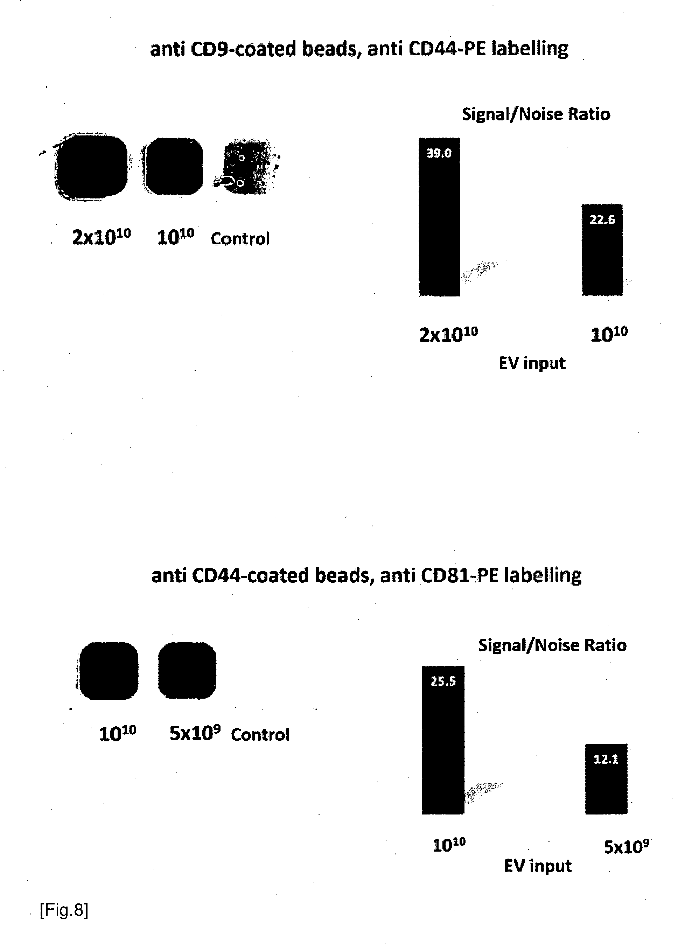

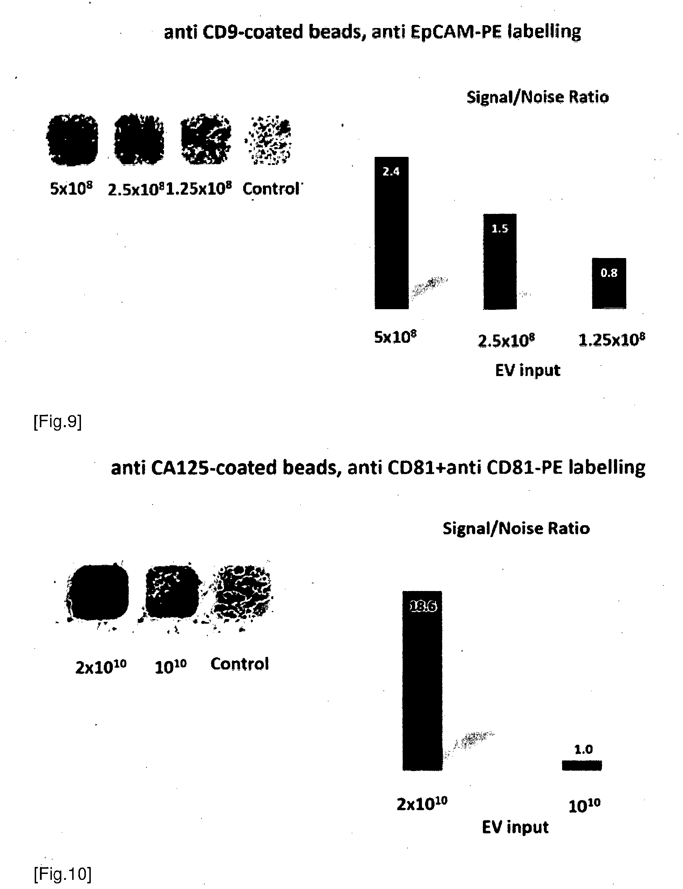

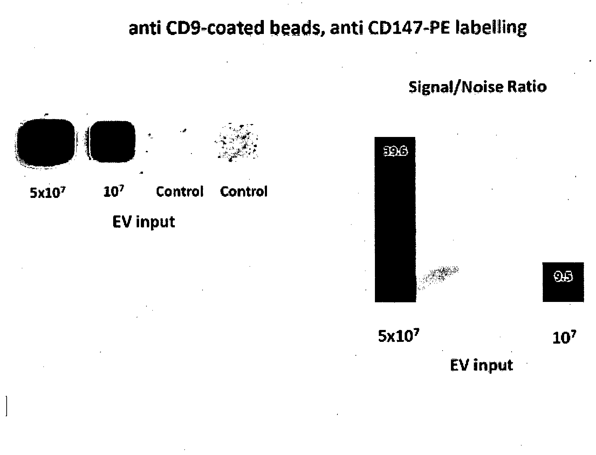

Figures 6-10 show application of 3D ELISA "in the volume" setup for profiling of mesenchymal markers CD166, CD44, EpCam, CD147 and oncomarker CA125 on small and middle HEK293t- and SKOV3-derived EVs.

## Claims

1. Method for the profiling of proteins from extracellular vesicles consisting of a 3D ELISA technique, **characterized in that** the 3D support are beads placed in microplate wells or microtubes or on the surface of the filter membrane and putted in contact with the extracellular vesicles.

2. Method for the profiling of proteins from extracellular vesicles according to the preceding claim, wherein said beads are magnetic or latex or polystyrene beads.

3. Method for the profiling of proteins from extracellular vesicles according to any one of claims 1 and 2, wherein the beads have a size between 50 nm and 5 µm.

4. Method for the profiling of proteins from extracellular vesicles according to any one of claims 1 to 3, wherein the pores of the filter membrane have a size between 5 kDa and 0.2 µm.

5. Method for the profiling of proteins from extracellular vesicles according to any one of claims 1 to 4, wherein the quantity of beads is between 10⁵ and 10⁸ per reaction.

6. Method for the profiling of proteins from extracellular vesicles according to any one of claims 1 to 5, wherein said beads are coated with crosslinking reagents that allows to immobilize the total extracellular vesicles population or to immobilize the entire set of proteins from lysed EVs.

7. Method for the profiling of proteins from extracellular vesicles according to any one of claims 1 to 6, wherein the beads are antibody-coated beads putted in contact with the extracellular vesicles, allowing the selection of total or specific extracellular population.

8. Method for the profiling of proteins from extracellular vesicles according to the preceding claim, wherein the antibody to at least one specific protein is chosen among: CD9 or CD81 or CD63 or CD44 or CD10 or CD166 or CD117 or CD66 or CD147 or CD24 or EGFR or EpCam or CD105 or NOTCH1 or NOTCH2 or NOTCH3 or NOTCH4 or CA125 or CA15-5 or CA19-9 or PSA or PDL1 or PDL2.

9. Method for the profiling of proteins from extracellular vesicles according to any of claims 1 to 8, comprising a step of removing the components which are unbound to the beads.

10. Method for the profiling of proteins from extracellular vesicles according to any one of claims 1 to 9, comprising a step of adding a labeling agent to visualize the beads-extracellular vesicles complexes.

11. Method for the profiling of proteins from extracellular vesicles according to the preceding claim, wherein the said labeling agent is chosen among:
- Fluorescent labeled antibodies to general or specific EV markers, preferably CD9 or CD81 or CD63 or CD44 or CD10 or CD166 or CD117 or CD66 or CD147 or CD24 or EGFR or EpCam or CD105 or NOTCH1 or NOTCH2 or NOTCH3 or NOTCH4 or CA125 or CA15-5 or CA19-9 or PSA or PDL1 or PDL2.
- Lipid dyes, preferably DIO or DIL or DID; or
- Nucleic acids staining dyes.

12. Method for the profiling of proteins from extracellular vesicles according to any of claims 1 to 11, comprising a step of removing the labeling agents which are unbound to the beads.

13. Method for the profiling of proteins from extracellular vesicles according to the preceding claim, comprising a step of registration for each sample in study of the total signal from the labeled EVs or cargo proteins attached to the beads.

14. Method for the profiling of proteins from extracellular vesicles according to any one of claims 1 to 13, wherein the extracellular vesicles are pre-purified before being putted in contact with the beads.

15. Method for the profiling of proteins from extracellular vesicles according to the preceding claim, wherein the membrane of the pre-purified extracellular vesicles is disrupted, preferably using a lysis buffer and/or ultrasound treatment, in order to analyze cargo proteins from the extracellular vesicles.

16. Method for the profiling of proteins from extracellular vesicles according to the preceding claim, wherein membrane and cargo proteins of the disrupted extracellular vesicles are immobilized on the beads coated with crosslinking reagent followed by immunostaining with labeled antibody to the marker(s) of interest. |
